# EUROPEAN PATENT APPLICATION

(11) **EP 4 506 025 A1**
(43) Date of publication of application: **12.02.2025**
(21) Application number: 23796709.6
(22) Date of filing: 21.04.2023
(51) Int. Cl.: A61M 5/145, A61M 5/172, A61M 5/142, G16H 20/17, G16H 50/20, G16H 40/63

(54) **DRUG INJECTION DEVICE AND METHOD**

(30) Priority: 28.04.2022 KR 20220052491; 23.08.2022 KR 20220105166; 20.04.2023 KR 20230051697
(71) Applicant: Caremedi Co., Ltd., Seoul 07207 (KR); Sogang University Research & Business Development Foundation, Seoul 04107 (KR)
(72) Inventor: SHIN, Woonsup, Seoul 04065 (KR); ZHU, Enhua, Seoul 04109 (KR); SHIN, Yeong Jong, Gimpo-si, Gyeonggi-do 10126 (KR)
(74) Representative: Manitz Finsterwald Patent- und Rechtsanwaltspartnerschaft mbB
(86) International application number: PCT/KR2023/005437
(87) International publication number: WO 2023/211062

(57) **Abstract**

A drug injection device includes a drug injector configured to electrochemically drive an electroosmotic pump to suck a drug from a drug storage and discharge an sucked drug to an injection target, and a controller configured to output, to the drug injector, a control signal corresponding to an injection sequence for defining an operation of the electroosmotic pump, wherein the injection sequence includes a pulse block that defines one of a voltage pulse and a current pulse to be applied to the electroosmotic pump, the pulse block defines a pair of pulse signals including a forward pulse and a reverse pulse that are applied to the electroosmotic pump to alternately generate negative pressure and positive pressure, and the electroosmotic pump sucks and discharges a drug by alternately generating the negative pressure and the positive pressure in the pulse block.

## Description

### BACKGROUND

The present disclosure relates to a drug injection device and a drug injection method.

Drugs may be injected into the body in various ways, such as oral, subcutaneous, and intravenous administration, depending on the type, purpose of treatment, and method. A drug injection device using a drug pump may automatically inject drugs into the body at a desired speed and dose at a required time. Therefore, a drug injection device using a drug pump may be used not only in hospitals or in patients' daily living environments, but also in various forms.

An insulin pump, which is commonly called an insulin injector, is a medical device that supplies insulin into the body from the outside at a set time to accurately control blood sugar levels, similar to a pancreas, for diabetic patients who do not secrete insulin or secrete only a small amount of insulin.

Such an insulin pump is used by insulin-dependent diabetic patients, and may continuously inject drugs for 24 hours in a state of being attached to the patient. In this way, an insulin injector has to periodically inject drugs for a long period of time for diabetic patients, and accordingly, active technology development is being conducted to miniaturize and automate insulin injectors for the convenience of users.

However, as insulin injectors are miniaturized and automated, there are cases where it is difficult to actually secure the stability of the operation of the insulin injector, and this is mostly due to the back pressure caused by various reactions between the drug and the biological fluid at the tip of the cannula or needle, which is the main cause of the blockage phenomenon. In particular, when the amount of injected drug is small or is injected slowly, the blockage phenomenon becomes more serious.

Therefore, a method of continuously inject drugs into patients, stably inject an accurate amount of drugs, and control the injection of drugs such that a path is not blocked during the drug injection is required.

### SUMMARY

The present disclosure provides a drug injection device and method that may inject a fixed amount of drug according to injection conditions.

However, technical tasks to be achieved by the present embodiments are not limited to the technical tasks described above, and there may be other technical tasks.

According to an embodiment of the present disclosure, a drug injection device includes a drug injector configured to electrochemically drive an electroosmotic pump to suck a drug from a drug storage and discharge an sucked drug to an injection target; and a controller configured to output, to the drug injector, a control signal corresponding to an injection sequence for defining an operation of the electroosmotic pump, wherein the injection sequence includes a pulse block that defines one of a voltage pulse and a current pulse to be applied to the electroosmotic pump, the pulse block defines a pair of pulse signals including a forward pulse and a reverse pulse that are applied to the electroosmotic pump to alternately generate negative pressure and positive pressure, and the electroosmotic pump sucks and discharges a drug by alternately generating the negative pressure and the positive pressure in the pulse block.

According to another embodiment of the present disclosure, a drug injection method of injecting a drug by using a drug injection device including an electroosmotic pump includes setting an injection sequence that determines an operation of the drug injection device, applying a control signal corresponding to the injection sequence to the electroosmotic pump, and sucking and discharging the drug by causing the electroosmotic pump to alternately generate negative pressure and positive pressure for each pulse block in response to the control signal, wherein the injection sequence includes a pulse block that defines one of a voltage pulse and a current pulse to be applied to the electroosmotic pump, and the pulse block defines a pair of pulse signals including a forward pulse and a reverse pulse that are applied to the electroosmotic pump to alternately generate negative pressure and positive pressure.

According to the present disclosure described above, ae drug injector may inject a fixed amount of drug by setting an appropriate injection sequence for the drug injector by using an electroosmotic pump.

In addition, it is possible to set an injection sequence for injecting a drug by using various data, and thus, usability according to a user and situation may be increased.

### BRIEF DESCRIPTION OF THE DRAWINGS

Embodiments of the inventive concept will be more clearly understood from the following detailed description taken in conjunction with the accompanying drawings in which:
FIG. 1 is a block diagram schematically illustrating a drug injection device according to an embodiment of the present disclosure;
FIG. 2 is a block diagram schematically illustrating a configuration of a drug injector illustrated in FIG. 1;
FIG. 3 illustrates concept of an injection sequence according to an embodiment of the present disclosure;
FIG. 4 is an example diagram illustrating an injection sequence according to an embodiment of the present disclosure;
FIG. 5 is a block diagram schematically illustrating a configuration of an electroosmotic pump illustrated in FIG. 2;
FIG. 6 is a block diagram schematically illustrating a configuration of a driver illustrated in FIG. 5;
FIG. 7 is an example diagram schematically illustrating an operation of the driver illustrated in FIG. 6;
FIG. 8 is an application example diagram of a drug injection device according to an embodiment of the present disclosure;
FIG. 9 is a block diagram schematically illustrating a configuration of the drug injection device illustrated in FIG. 8;
FIG. 10 is a block diagram schematically illustrating a configuration of the drug injection device illustrated in FIG. 8;
FIG. 11 is an example diagram of a table in which multiple injection sequences are stored;
FIG. 12 is an example diagram illustrating information on multiple pulse blocks; and
FIG. 13 is a flowchart illustrating a drug injection method according to an embodiment of the present disclosure.

### DETAILED DESCRIPTION OF THE EMBODIMENTS

Hereafter, the present disclosure will be described in detail with reference to the accompanying drawings. However, the present disclosure may be implemented in many different forms and is not limited to the embodiments described herein. In addition, the accompanying drawings are only for easy understanding of the embodiments disclosed in the present specification, and the technical ideas disclosed in the present specification are not limited by the accompanying drawings. In order to clearly describe the present disclosure in the drawings, parts irrelevant to the descriptions are omitted, and a size, a shape, and a form of each component illustrated in the drawings may be variously modified. The same or similar reference numerals are assigned to the same or similar portions throughout the specification.

Suffixes "module" and "unit" for the components used in the following description are given or used interchangeably in consideration of ease of writing the specification, and do not have meanings or roles that are distinguished from each other by themselves. In addition, in describing the embodiments disclosed in the present specification, when it is determined that a detailed descriptions of related known technologies may obscure the gist of the embodiments disclosed in the present specification, the detailed descriptions are omitted.

Throughout the specification, when a portion is said to be "connected (coupled, in contact with, or combined)" with another portion, this includes not only a case where it is "directly connected (coupled, in contact with, or combined)" ", but also a case where there is another member therebetween. In addition, when a portion "includes (comprises or provides)" a certain component, this does not exclude other components, and means to "include (comprise or provide)" other components unless otherwise described.

Terms indicating ordinal numbers, such as first and second, used in the present specification are used only for the purpose of distinguishing one component from another component and do not limit the order or relationship of the components. For example, the first component of the present disclosure may be referred to as the second component, and similarly, the second element may also be referred to as the first component.

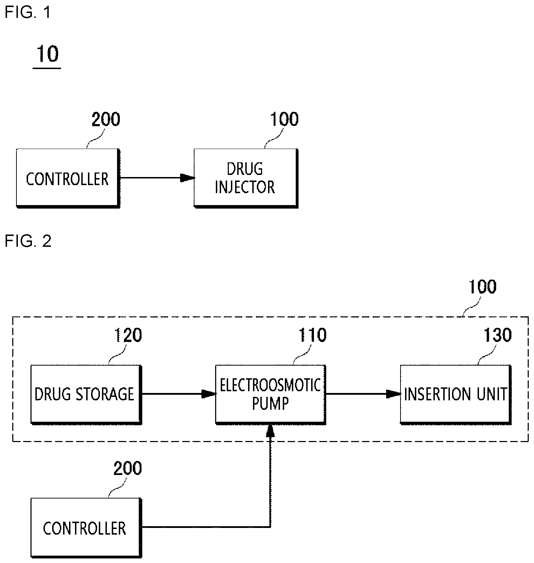

FIG. 1 is a block diagram schematically illustrating a configuration of a drug injection device according to an embodiment of the present disclosure, and FIG. 2 is a block diagram schematically illustrating a configuration of the drug injection device illustrated in FIG. 1.

Referring to FIG. 1 and FIG. 2, a drug injection device 10 according to an embodiment of the present disclosure includes a drug injector 100 and a controller 200.

The drug injector 100 electrochemically drives an electroosmotic pump 110 to suck a drug from a drug source and discharges the sucked a drug to an injection target. Then, the controller 200 outputs a control signal corresponding to an injection sequence that defines an operation of the electroosmotic pump 110 to the drug injector 100.

The controller 200 may mean a data processing device which is embedded in hardware and includes a physically structured circuit to perform a function expressed as a code or command included in a program. The data processing device embedded in hardware may include, for example, a microprocessor, a central processing unit (CPU), a processor core, a multiprocessor, an application-specific integrated circuit (ASIC), a field programmable gate array (FPGA), a micro control unit (MCU), an embedded processor, or so on, but the scope of the present disclosure is not limited thereto.

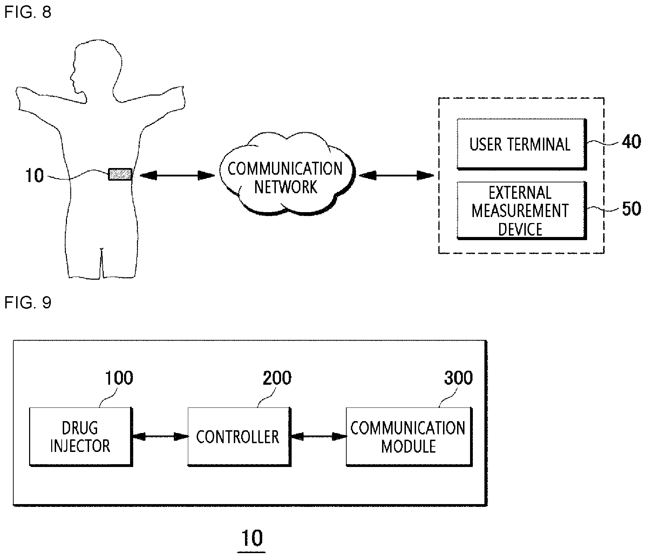

Meanwhile, the controller 200 may be embedded solely in the drug injection device 10, or may control an operation of the drug injection device 10 by being connected to a user terminal 40 (see FIG. 8) through a communication module and being integrated into the user terminal 40.

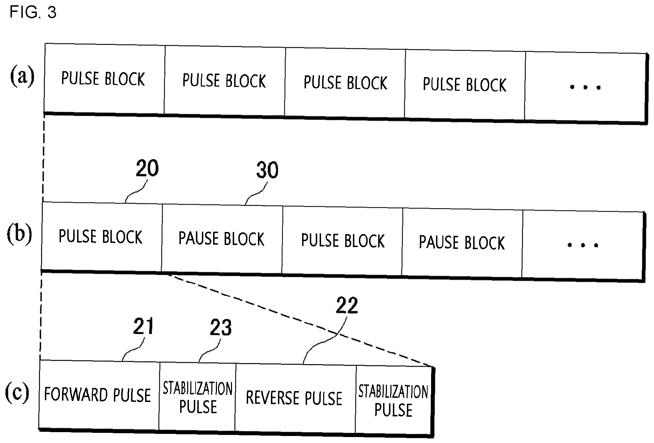

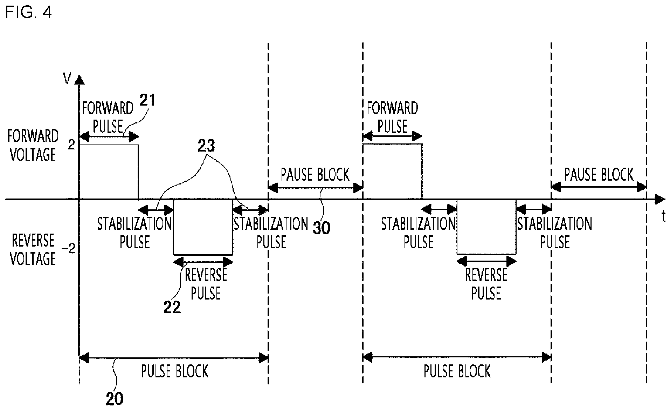

FIG. 3 illustrates the concept of an injection sequence according to an embodiment of the present disclosure, and FIG. 4 is an example diagram illustrating the injection sequence according to the embodiment of the present disclosure. Hereinafter, an injection sequence will be described in detail with reference to FIGS. 3 and 4.

As illustrated in (a) of FIG. 3, the injection sequence includes one or more pulse blocks 20, which are sequentially arranged, to be applied to the electroosmotic pump 110. In addition, as illustrated in (c) of FIG. 3, each of the pulse blocks 20 includes information on a pair of pulse signals including a forward pulse and a reverse pulse, and includes information on an amplitude of each pulse and a duration for which a pulse is maintained. The pair of pulse signals including the forward pulse and the reverse pulse are applied to the electroosmotic pump 110 to alternately generate negative pressure and positive pressure, and thereby, the negative pressure and positive pressure are alternately generated in each of the pulse blocks 20 to suck and discharge a drug.

A pair of pulse signals 21 and 22 included in the pulse block 20 is a voltage pulse signal or a current pulse signal. A pair of voltage pulse signals includes a forward voltage pulse and a reverse voltage pulse, and a pair of current pulse signals includes a forward current pulse and a reverse current pulse. Here, the pair of voltage pulse signals may include information on amplitudes and durations of the respective voltage pulses, and the pair of current pulse signals may include information on amplitudes and durations of the respective current pulses. For example, in the injection sequence illustrated in FIG. 4, the amplitude of a pulse signal may be 2 volts, and the duration thereof may be 10 seconds.

Pulse blocks included in the injection sequence may each include voltage pulses having different voltage levels or current pulses having different current levels. Alternatively, the pulse blocks may each include pulses having the same voltage level or current pulses having the same current level, but respective durations may be set differently.

In addition, the forward pulse 21 and the reverse pulse 22 included in the pulse block 20 may be set to have the same amplitude and duration, and accordingly, amounts of drugs sucked and discharged by the electroosmotic pump 110 may be maintained to be the same.

In addition, the pair of pulse signals 21 and 22 included in the pulse block 20 may be provided as a constant voltage or a constant current, and the amount of drugs sucked and discharged by each pulse block may be adjusted by controlling the duration of a pulse signal provided by the constant voltage or the constant current. For example, the pulse blocks 20 of FIG. 4 are provided by a constant voltage of 2 volts.

Additionally, the pair of pulse signals 21 and 22 may be controlled in both amplitudes and durations, and accordingly, the amounts of drugs sucked and discharged may be adjusted to be the same. For example, an amplitude of the forward voltage pulse is 2 volts, the duration is 10 seconds, and the amplitude of a subsequent reverse voltage pulse is set to 1 volt, and the duration thereof is 20 seconds, and accordingly, an area of the forward pulse and an area of the reverse pulse are set to equal to each other to maintain the amount of sucked drug and the amount of discharged drug to be equal to each other.

In addition, referring to FIG. 4, the pulse block 20 may further include a stabilization pulse 23, and the stabilization pulse 23 is a pulse that maintains a voltage of 0 volt for a predetermined time after the forward voltage pulse 21 and the reverse voltage pulse 22 are applied. An operation of the electroosmotic pump 110 may be stabilized by the stabilization pulse 23. Here, when the pulse block 10 of an injection sequence includes a pair of current pulses, the pulse block 20 may further include the stabilization pulse 23 that maintains a current of 0 ampere for a predetermined time after the forward current pulse and the reverse current pulse are applied.

Additionally, as illustrated in (b) of FIG. 3, the injection sequence may further include a pause block 30. The pause block 30 is provided after the pulse block 20 is applied or is provided between the pulse blocks 20 and is a signal in which a voltage of 0 volt or a current of 0 ampere is maintained for a predetermined time. The drug injector 100 of the present disclosure has to slowly inject a certain amount of drug for a long time. Therefore, as the injection sequence includes the pause block 30, the time at which a target drug injection amount is injected may be adjusted, and by minimizing a time period during which a drug is not injected, it is possible to prevent a flow path of the drug injector 100 from being blocked during a time period during which a drug is not injected.

The controller 200 generates a control signal including a voltage pulse or a current pulse corresponding to an injection sequence based on information of a pulse block included in the injection sequence and applies the control signal to the drug injector 100.

When a control signal corresponding to the injection sequence is applied to the electroosmotic pump 110, the electroosmotic pump 110 alternately generates negative pressure and positive pressure for each pulse block to suck and discharge drugs. Here, the control signal may be a signal including a voltage pulse pair including a forward voltage pulse and a reverse voltage pulse in units of pulse blocks or a current pulse pair including a forward current pulse and a reverse current pulse in units of pulse blocks.

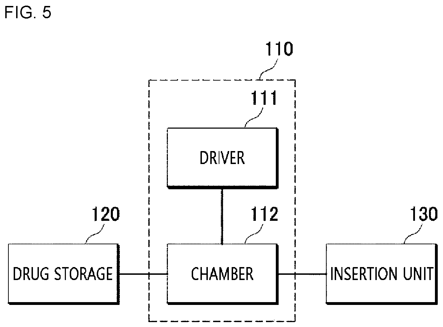

FIG. 5 is a block diagram schematically illustrating a configuration of the electroosmotic pump 110 illustrated in FIG. 2.

Referring to FIG. 5, the electroosmotic pump 110 includes a driver 111 and a chamber 112. The driver 111 is electrochemically driven by a control signal to generate positive pressure and negative pressure and discharges a drug according to the positive pressure and negative pressure, and the chamber 112 sucks a drug from a drug storage 120 according to the pressure of the driver 111 and then discharges the drug to an insertion unit 130. Here, the drug is a drug that has to be injected into a certain patient and may be, for example, insulin that is injected into a diabetic patient.

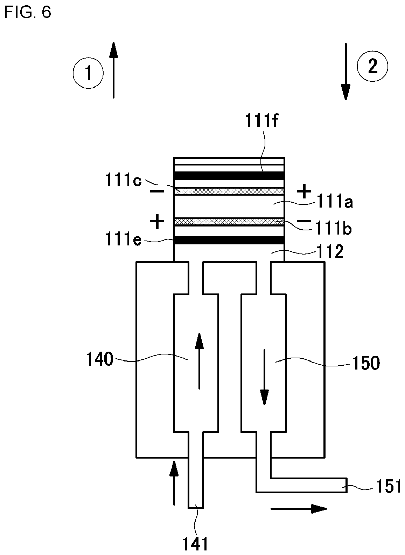

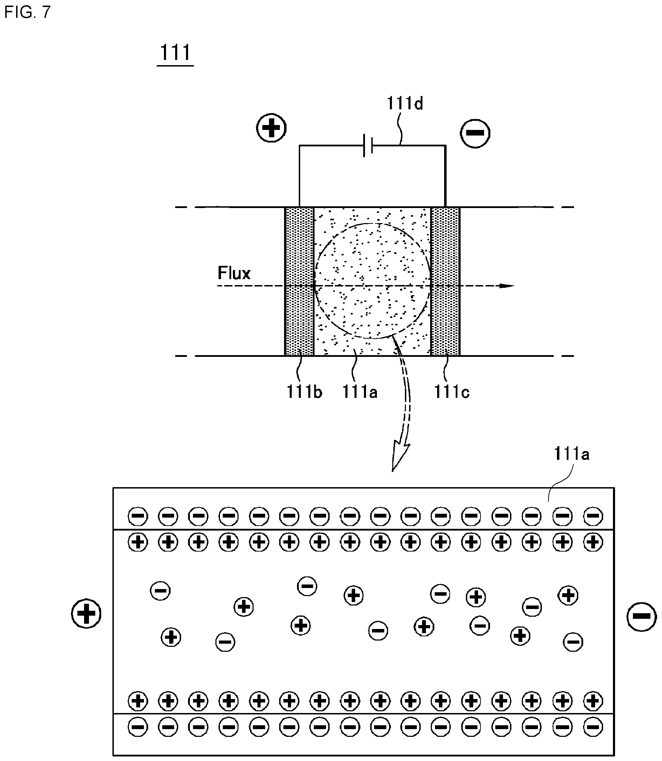

FIG. 6 is a block diagram schematically illustrating a configuration of the driver 111 illustrated in FIG. 5, and FIG. 7 is an example diagram schematically illustrating a configuration of the driver 111 illustrated in FIG. 6.

Referring to FIGS. 6 and 7, the driver 111 is a pump that uses the movement of fluid according to an electroosmotic phenomenon that occurs when a voltage or current is applied by using electrodes at both ends of a porous film (membrane) 111a. The driver 111 may include the membrane 111a, a power source 111d that applies a voltage or current to a first electrode 111b and a second electrode 111c arranged on both sides of the membrane 111a, and a flow path through which a fluid moves.

In addition, the driver 111 may include a first diaphragm 111e arranged adjacent to the first electrode 111b and a second diaphragm 111f arranged adjacent to the second electrode 111c. The first and second diaphragms 111eand 111f are provided respectively one side and the other side of the membrane 111a, and shapes of the first and second diaphragms 111e and 111f are deformed by the movement of a pumping solution as positive pressure and negative pressure are alternately generated. For example, the first diaphragm 111e and the second diaphragm 111f transfer the negative pressure and positive pressure generated by an operation of the membrane 111a to a transfer target fluid. More specifically, when negative pressure is generated, at least a part of the first diaphragm 111e and the second diaphragm 111f moves backward (moving in a direction ①) such that the transfer target fluid is sucked to the chamber 112, and in contrast to this, when positive pressure is generated, at least a part of the first diaphragm 111e and the second diaphragm 111f moves forward (moving in a direction ②) such that the transfer target fluid is discharged from the chamber 112.

Silica, glass, and so on are generally used as a material of the porous film 111a, and when the silica and glass are immersed in an aqueous solution, surfaces thereof are negatively charged. There are many paths, through which fluids may pass, in the porous film 111a, and when one of the paths is enlarged, a surface of the fluid path with negative charges (bound anions) may be in a pseudo-state in which the negative charges are balanced by mobile cations having positive charges. In this state, when a positive voltage is applied to the first electrode 111b and a negative voltage is applied to the second electrode 111c, negative pressure is generated, and the fluid inside the driver 111 moves in the direction ① due to the negative pressure. In this case, a drug is sucked through an inflow path 141 and flows into the chamber 112 through an inflow valve 140. In this case, an outflow valve 150 is closed such that the negative pressure is not transferred to an outflow path 151.

In contrast to this, when a negative voltage is applied to the first electrode 111b and a positive voltage is applied to the second electrode 111c, the positive pressure in an opposite direction is generated by a reversible electrochemical reaction. The fluid inside the driver 111 moves in the direction ② by the positive pressure. In this case, the drug stored in the chamber 112 is introduced into a target through the outflow valve 150 and the outflow path 151. In this case, the inflow valve 140 is blocked such that the positive pressure is not transferred to the inflow path 141. Meanwhile, the inflow valve 140 and the outflow valve 150 are check valves that move a fluid in one direction and prevent a backflow of the fluid when pressure is released.

This phenomenon is called an electroosmotic phenomenon, and a pump that uses the principle is the electroosmotic pump 110. Therefore, the pressure generated by the electroosmotic pump 110 and a volume of the drug discharged from the electroosmotic pump 110 can be adjusted by controlling amplitudes and application times of voltages or currents applied to the first and second electrodes 111b and 111c.

According to the configuration, polarities of a voltage or current are alternately supplied to the first electrode 111b and the second electrode 111cof the driver 111, and thereby, reversible electrochemical reactions occur in the forward and reverse directions. As the forward and reverse electrochemical reactions occur repeatedly, the fluid inside the electroosmotic pump 110 repeatedly performs a reciprocating motion. In addition, the first electrode 111b and the second electrode 111c are repeatedly consumed and regenerated by the repetitive forward and reverse reversible electrochemical reactions. When the inflow valve 140 and the outflow valve 150 are coupled to the chamber 112 of the electroosmotic pump 110, during the inflow operation, the drug in the drug storage 120 is sucked into the inflow path 141 and stored in the chamber 112 through the inflow valve 140. In addition, during the outflow operation, the drug stored in the chamber 112 is discharged to the insertion unit 130 through the outflow valve 150 and the outflow path 151.

An electrode used for the driver 111 may be implemented by a platinum mesh such as a porous electrode, porous carbon paper or fiber, or various electrode materials applied onto a porous structure so as to smoothly move a fluid.

In addition, the electrode used for the driver 111 may be coated with various fixable materials, such as a non-transparent base material, by drop coating or spin coating. In this case, the non-transparent base material is a plate-shaped substrate including at least one of a conductive material, a semiconductor material, and a non-conductive material, and an electrode material applied thereon may include metal, metal oxide, conducting polymer, metal hexacyanoferrate, a carbon nanostructure, or a composite thereof.

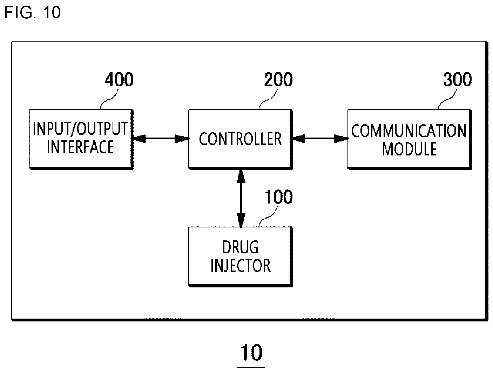

FIG. 8 is an application example diagram of a drug injection device according to an embodiment of the present disclosure, FIG. 9 is a block diagram schematically illustrating a configuration of the drug injection device illustrated in FIG. 8, and FIG. 10 is a block diagram schematically illustrating the configuration of the drug injection device illustrated in FIG. 8. An operation of the drug injection device 10 will be specifically described with reference to FIGS. 8 to 10.

The drug injection device 10 receives predetermined information from a user and sets an injection sequence based on the information, and a process of setting the injection sequence may be performed according to data used to set the injection sequence. Hereinafter, the process of setting the injection sequence will be described.

The process of setting, by the drug injection device 10, the injection sequence according to an embodiment of the present disclosure will be described with reference to FIG. 9.

The drug injection device 10 may set the injection sequence by using a drug injection condition. Here, the drug injection condition includes a drug injection amount, a drug injection period and drug injection speed, or the drug injection amount and the drug injection period. A drug injection condition of a diabetic patient may be set differently depending on basal injection and immediate injection, and the basal injection is an injection method of maintaining a constant blood sugar level of a diabetic patient by injecting a drug at a set speed for a set period of time, and the immediate injection is an injection method for injecting a drug to a diabetic patient without a rest period. The basal injection and immediate injection may be adjusted according to a user's choice.

The drug injection device 10 may further include a communication module 300 to receive a drug injection condition from the user terminal 40. The drug injection condition is received from a user through the user terminal 40 that is communicably connected to the drug injection device 10 through the communication module 300. Then, the controller 200 sets an injection sequence corresponding to the drug injection condition and transmits a control signal to the drug injector 100. Here, the injection sequence is not set by using the drug injection condition but may be directly selected by a user through the user terminal 40 or may be received by being calculated by an external computing device based on the user's past use history of the drug injection device.

The user terminal 40 may include a computer or portable terminal that may be connected to the drug injection device 10 through wireless communication. Here, the computer includes, for example, a desktop computer, a laptop computer, or so on in which a web browser is stored, and the portable terminal is, for example, a wireless communication device that ensures portability and mobility and may include all kinds of handheld-based wireless communication devices, such as various smartphones, tablet personal computers (PCs), and smart watches. The user terminal 40 is managed by a wearer of the drug injection device 10 or a medical professional, and may set a drug injection condition through an application running on the user terminal 40.

In addition, the drug injection device 10 may set the injection sequence by using user input data. Here, the user input data includes a user's blood sugar level or the user's meal information. The user input data is received from a user through the user terminal 40 connected to the drug injection device 10 through the communication module 300, and the controller 200 calculates the drug injection condition based on the received user input data. In addition, an injection sequence corresponding to the drug injection condition is set, and a control signal corresponding to the injection sequence is transmitted to the drug injector 100.

In addition, the drug injection device 10 may set the injection sequence by using user biometric data. Here, the user biometric data includes a user's blood sugar information. The user biometric data is received through an external measurement device 50 connected to the drug injection device 10 through the communication module 300, and the controller 200 calculates a drug injection condition based on the received user biometric data. In addition, an injection sequence corresponding to the drug injection condition is set, and a control signal corresponding to the injection sequence is transmitted to the drug injector 100. Here, the external measurement device 50 may be a device that senses a user's blood sugar level.

Next, a process of setting the injection sequence by the drug injection device will be described with reference to FIG. 10.

The drug injection device 10 may set an injection sequence by using a drug injection condition. The drug injection device 10 may further include a user input/output interface module 400, and receives a drug injection condition from a user through the user input/output interface module 400. In an embodiment in which the drug injection device 10 includes the user input/output interface module 400, a user may control an operation of the drug injection device 10 by directly inputting a drug injection condition and so on to the user input/output interface module 400 without separately using the user terminal 40. In this case, the communication module 300 may be optionally excluded.

The controller 200 sets an injection sequence corresponding to the drug injection conditions input through the user input/output interface module 400 and transmits a control signal corresponding to the injection sequence to the drug injector 100. Here, the injection sequence may be directly selected by a user through the user input/output interface module 400 without being set by using the drug injection condition.

The user input/output interface module 400 may include a physical input/output button coupled to an external housing including the drug injection device 10, and a signal processing circuit that transmits a signal generated according to an operation of the physical input/output button to the controller 200.

Alternatively, the user input/output interface module 400 may output a user interface (UI) that guides a user to input information on a drug injection condition or injection sequence through a display including a touch screen.

Additionally, in the process of setting the injection sequence described above, the controller 200 may also set an injection sequence corresponding to each drug injection condition by referring to a table in which multiple injection sequences are stored for each drug injection condition.

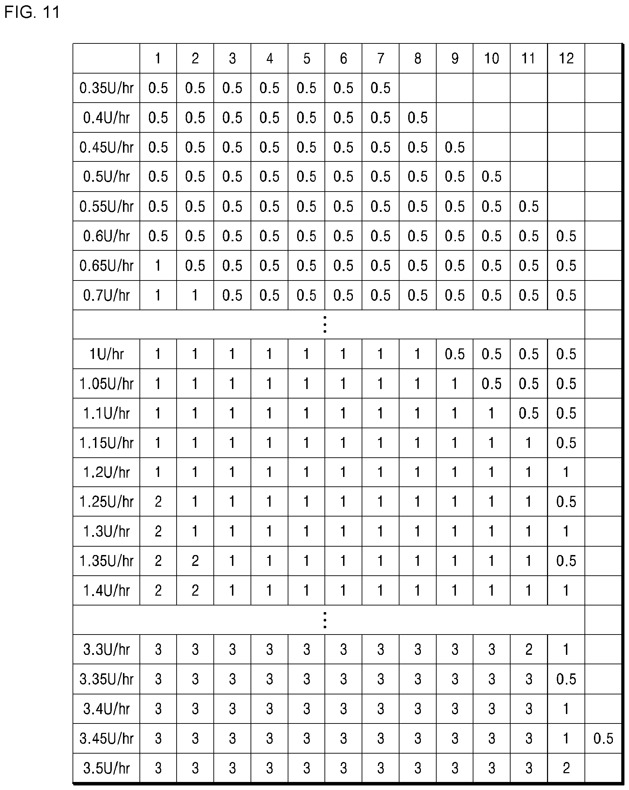

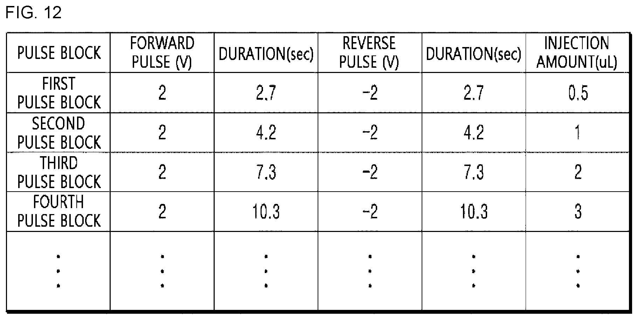

FIG. 11 is an example diagram of a part of a table in which multiple injection sequences are stored, and FIG. 12 is an example diagram illustrating information on multiple pulse blocks. The controller 200 may set by matching an injection sequence corresponding to each drug injection condition in the table illustrated in FIG. 11. That is, the injection sequence may be set according to a drug injection condition, such as an injection speed or an injection amount.

A process of setting an injection sequence according to a drug injection condition will be described as an example with reference to FIG. 11 and FIG. 12. Referring to FIG. 11, when the injection speed (injection amount per hour) is input as a drug injection condition, the controller 200 sets an injection sequence corresponding to the input injection speed. When 1 U/hr is input as the drug injection condition, the controller 200 sets the injection sequence set to 1 U/hr as the injection sequence for the drug injection condition. The injection sequence set to 1 U/hr is composed of 8 pulse blocks supplying 1 µL and 4 pulse blocks supplying 0.5 µL.

Referring to FIG. 12, the injection sequence includes a combination of M pulse blocks that satisfy the drug injection speed among N pulse blocks supplying different amounts of drugs. For example, the injection sequence set to 1 U/hr as illustrated in FIG. 11 is structured such that the second pulse block is arranged 8 times, and then the first pulse block is arranged 4 times. Therefore, the injection sequence may be configured with different pulse blocks depending on drug injection conditions. Although FIG. 12 illustrates that pulse blocks include forward voltage pulses and reverse voltage pulses, the pulse blocks are not limited thereto and may be composed of forward current pulses and reverse current pulses in some cases.

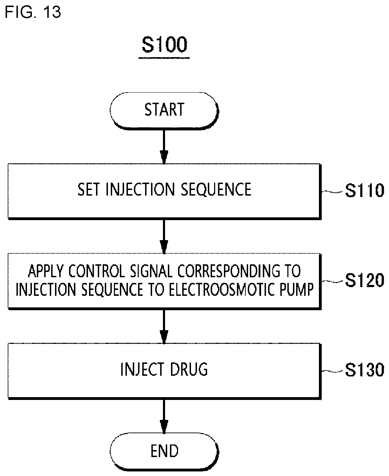

FIG. 13 is a flowchart illustrating a drug injection method according to an embodiment of the present disclosure.

Referring to FIGS. 1, 2, and 13, a drug injection method S100 according to an embodiment of the present disclosure includes step S110 of setting an injection sequence for determining an operation of the drug injection device 10 and step S120 of applying a control signal corresponding to the injection sequence to the electroosmotic pump 110. Thereafter, in response to the control signal, the electroosmotic pump 110 alternately generates negative pressure and positive pressure for each pulse block to suck and discharge a drug and inject the drug into a user (step S130). Here, the injection sequence includes at least one pulse block defining a voltage pulse or current pulse to be applied to the electroosmotic pump 110, and each pulse block defines a pair of pulse signals including a forward pulse and a reverse pulse that are applied to the electroosmotic pump 110 to alternately generate the negative pressure and positive pressures.

Hereinafter, respective steps will be described in detail.

A process of setting the injection sequence (step S110) may be divided into various embodiments according to data used to set the injection sequence.

First, the drug injection device 10 may set the injection sequence by using a drug injection condition. Here, the drug injection condition includes a drug injection amount, a drug injection period and a drug injection speed, or the drug injection amount and the drug injection period. The drug injection device 10 may receive the drug injection condition from a user through the user terminal 40 connected to the drug injection device 10 through the communication module 300. Then, the controller 200 sets an injection sequence corresponding to the drug injection condition and transmits a control signal corresponding the injection sequence to the drug injector 100. Here, the injection sequence may be directly selected by a user through the user terminal 40 without being set by using the drug injection condition.

In addition, the drug injection device 10 may receive the drug injection condition from the user through the user input/output interface module 400, and the controller 200 sets an injection sequence corresponding to the drug injection condition and transmits a control signal to the drug injector 100. Here, the injection sequence may be directly selected by a user through the user input/output interface module 400 without being set by using the drug injection condition.

In addition, the drug injection device 10 may set the injection sequence by using user input data. Here, the user input data includes a user's blood sugar level or a user's meal information. The user input data is received from a user through the user terminal 40 communicatively connected to the drug injection device 10 through the communication module 300, and the controller 200 calculates the drug injection condition based on the received user input data. In addition, the injection sequence corresponding to the drug injection condition is set, and the control signal corresponding to the injection sequence is transmitted to the drug injector 100.

In addition, the drug injection device 10 may set the injection sequence by using a user's biometric data. Here, the user's biometric data includes a user's blood sugar level information. The user's biometric data is received through the external measurement device 50 communicatively connected to the drug injection device 10 through the communication module 300, and the controller 200 calculates the drug injection condition based on the received user's biometric data. In addition, the injection sequence corresponding to the drug injection condition is set, and the control signal corresponding to the injection sequence is transmitted to the drug injector 100. Here, the external measurement device 50 may be a device that senses a user's blood sugar level.

Meanwhile, in the process of setting the injection sequence, the controller 200 may set the injection sequence corresponding to the drug injection condition by referring to a table in which multiple injection sequences are stored for each drug injection condition.

In addition, in a process of applying a control signal to the electroosmotic pump (step S120), the control signal corresponding to an injection sequence may include a voltage pulse pair including a forward voltage pulse and a reverse voltage pulse or a current pulse pair including a forward current pulse and a reverse current pulse in pulse block units, and in response to the control signal, the electroosmotic pump 110 may perform an operation of alternately generating negative pressure and positive pressure for each pulse block to suck and discharge a drug (step S130), thereby injecting the drug into a user.

A method according to an embodiment of the present disclosure may be performed in the form of a recording medium including instructions executable by a computer, such as a program module executed by a computer. A computer readable medium may be any available medium that may be accessed by a computer and includes both volatile and nonvolatile media, removable and non-removable media. Also, the computer readable medium may include a computer storage medium. A computer storage medium includes both volatile and nonvolatile media and removable and non-removable media implemented by any method or technology for storing information, such as computer readable instructions, data structures, program modules or other data.

In addition, although the method and system of the present disclosure are described with respect to specific embodiments, some or all of components or operations thereof may be implemented by using a computer system having a general-purpose hardware architecture.

those skilled in the art to which the present disclosure belongs will understand that the present disclosure may be easily modified into another specific form based on the descriptions given above without changing the technical idea or essential features of the present disclosure. Therefore, the embodiments described above should be understood as illustrative in all respects and not limiting. The scope of the present disclosure is indicated by the claims described below, and all changes or modified forms derived from the meaning, scope of the claims, and their equivalent concepts should be interpreted as being included in the scope of the present disclosure.

The scope of the present application is indicated by the claims described below rather than the detailed description above, and all changes or modified forms derived from the meaning, scope of the claims, and their equivalent concepts should be interpreted as being included in the scope of the present application.

## Claims

1. A drug injection device comprising:
a drug injector configured to electrochemically drive an electroosmotic pump to sucksuck a drug from a drug storage and discharge an sucked drug to an injection target; and
a controller configured to output, to the drug injector, a control signal corresponding to an injection sequence for defining an operation of the electroosmotic pump,
wherein the injection sequence includes one or more pulse blocks that defines one of a voltage pulse and a current pulse to be applied to the electroosmotic pump,
the pulse block defines a pair of pulse signals including a forward pulse and a reverse pulse that are applied to the electroosmotic pump to alternately generate negative pressure and positive pressure, and
the electroosmotic pump sucks and discharges a drug by alternately generating the negative pressure and the positive pressure in the pulse block.

2. The drug injection device of claim 1, wherein
an amplitude and a duration of the forward pulse included in the pair of pulse signals are set to be respectively equal to an amplitude and a duration of the reverse pulse included in the pair of pulse signals such that an amount of a drug sucked by the electroosmotic pump is equal to an amount of a drug discharged by the electroosmotic pump.

3. The drug injection device of claim 1, wherein
information on any one of a pair of voltage pulse signals and a pair of current pulse signals includes information on any one of an amplitude and duration of a voltage pulse and an amplitude and duration of a current pulse.

4. The drug injection device of claim 1, wherein
an amplitude and a duration of a pair of voltage pulse signals or an amplitude and a duration of a pair of current pulse signals supplied by the drug injection device are adjusted such that an amount of a drug sucked by any one of the pair of voltage pulse signals and the pair of current pulse signals is equal to an amount of a drug discharged by any one of the pair of voltage pulse signals and the pair of current pulse signals.

5. The drug injection device of claim 1, wherein
the drug injection device provides any one of a constant voltage and a constant current, and adjusts an amount of drugs sucked and discharged by the pulse block by adjusting any one of a duration of a voltage pulse and a duration of a current pulse provided by any one of the constant voltage and the constant current.

6. The drug injection device of claim 1, wherein
the pair of pulse signals includes a pair of voltage pulses including a forward voltage pulse and a reverse voltage pulse, and includes a stabilization pulse that maintains a voltage of 0 volt for a predetermined time after the forward voltage pulse and the reverse voltage pulse are applied, or
the pair of pulse signals includes a pair of current pulses including a forward current pulse and a reverse current pulse, and includes a stabilization pulse that maintains a current of 0 ampere for a predetermined time after the forward current pulse and the reverse current pulse are applied.

7. The drug injection device of claim 1, wherein
the injection sequence includes at least one pulse block, and includes a pause block that maintains any one of a voltage of 0 volt and a current of 0 ampere for a predetermined time after the pulse block is applied, or between adjacent pulse blocks.

8. The drug injection device of claim 1, wherein
the injection sequence includes multiple pulse blocks, and the multiple pulse blocks are arranged sequentially.

9. The drug injection device of claim 1, wherein the electroosmotic pump includes:
a driver configured to alternately generate the positive pressure and the negative pressure by being electrochemically driven by the control signal; and
a chamber configured to suck the drug from a drug source according to pressure of the driver and then discharge the drug to an insertion unit according to the pressure of the driver.

10. The drug injection device of claim 1, wherein
the drug injection device receives a drug injection condition from any one of a user terminal and a user input/output interface module,
the controller outputs the control signal by using an injection sequence corresponding to the drug injection condition, and
the drug injection condition includes any one of a drug injection amount, a drug injection period and a drug injection speed, and the drug injection amount and the drug injection period.

11. The drug injection device of claim 1, wherein
the drug injection device receives user input data from a user terminal,
the controller calculates a drug injection condition based on the user input data and outputs the control signal by using an injection sequence corresponding to the drug injection condition,
the user input data includes a blood sugar level of a user and any one of activity information and meal information of the user, and
the drug injection condition includes any one of a drug injection amount, a drug injection period and a drug injection speed, and the drug injection amount and the drug injection period.

12. The drug injection device of claim 1, wherein
the drug injection device receives user biometric data from an external measurement device,
the controller calculates a drug injection condition based on the user biometric data and outputs the control signal by using an injection sequence corresponding to the drug injection condition,
the user biometric data includes any one of user blood sugar level information and user activity information, and
the drug injection condition includes any one of a drug injection amount, a drug injection period and a drug injection speed, and the drug injection amount and the drug injection period.

13. The drug injection device of according to any one of claims 10 to 12, wherein
the controller sets the injection sequence corresponding to the drug injection condition by referring to a table in which multiple injection sequences are stored for each drug injection condition.

14. The drug injection device of claim 1, wherein
the drug injection device receives an injection sequence set by any one of a user terminal and a user input/output interface module, and
the controller outputs the control signal by using the injection sequence.

15. The drug injection device of claim 1, wherein
the injection sequence is received by being calculated by an external computing device based on a user's past use history of the drug injection device.

16. A drug injection method of injecting a drug by using a drug injection device including an electroosmotic pump, the drug injection method comprising:
setting an injection sequence that determines an operation of the drug injection device;
applying a control signal corresponding to the injection sequence to the electroosmotic pump; and
sucking and discharging the drug by causing the electroosmotic pump to alternately generate negative pressure and positive pressure for each pulse block in response to the control signal,
wherein the injection sequence includes a pulse block that defines one of a voltage pulse and a current pulse to be applied to the electroosmotic pump, and
the pulse block defines a pair of pulse signals including a forward pulse and a reverse pulse that are applied to the electroosmotic pump to alternately generate negative pressure and positive pressure.

17. The drug injection method of the drug injection device of claim 16, wherein,
in the setting of the injection sequence, an amplitude and a duration of the forward pulse included in the pair of pulse signals are set to be respectively equal to an amplitude and a duration of the reverse pulse included in the pair of pulse signals such that an amount of a drug sucked by the electroosmotic pump is equal to an amount of a drug discharged by the electroosmotic pump.

18. The drug injection method of the drug injection device of claim 16, wherein
the pulse block included in the injection sequence includes information on any one of a pair of voltage pulse signals including a forward voltage pulse and a reverse voltage pulse and a pair of current pulse signals including a forward current pulse and a reverse current pulse.

19. The drug injection method of the drug injection device of claim 18, wherein
information on any one of a pair of voltage pulse signals and a pair of current pulse signals includes information on any one of an amplitude and duration of a voltage pulse and an amplitude and duration of a current pulse.

20. The drug injection method of the drug injection device of claim 18, wherein,
in the setting of the injection sequence, amounts of drugs sucked and discharged according to any one of the pair of voltage pulse signals and the pair of current pulse signals are set to be equal to each other by adjusting amplitudes and durations of the pair of voltage pulse signals or amplitudes and durations of the pair of current pulse signals.

21. The drug injection method of the drug injection device of claim 18, wherein,
in the setting of the injection sequence, an amount of drugs sucked and discharged by the pulse block is set to be adjusted by adjusting any one of a duration of a voltage pulse and a duration of a current pulse provided by any one of a constant voltage and a constant current supplied by the drug injection device.

22. The drug injection method of the drug injection device of claim 18, wherein
the pair of pulse signals includes a pair of voltage pulses including a forward voltage pulse and a reverse voltage pulse, and includes a stabilization pulse that maintains a voltage of 0 volt for a predetermined time after the forward voltage pulse and the reverse voltage pulse are applied, or
the pair of pulse signals includes a pair of current pulses including a forward current pulse and a reverse current pulse, and includes a stabilization pulse that maintains a current of 0 ampere for a predetermined time after the forward current pulse and the reverse current pulse are applied.

23. The drug injection method of the drug injection device of claim 16, wherein
the injection sequence includes a pause block that maintains any one of a voltage of 0 volt and a current of 0 ampere for a predetermined time after the pulse block is applied, or between adjacent pulse blocks.

24. The drug injection method of the drug injection device of claim 16, wherein
the injection sequence includes multiple pulse blocks, and the multiple pulse blocks are arranged sequentially.

25. The drug injection method of the drug injection device of claim 16, wherein,
in the setting of the injection sequence, the drug injection device sets the injection sequence based on a drug injection condition received from any one of a user terminal and a user input/output interface module, and
the drug injection condition includes any one of a drug injection amount, a drug injection period and a drug injection speed, and the drug injection amount and the drug injection period.

26. The drug injection method of the drug injection device of claim 16, wherein,
in the setting of the injection sequence, the drug injection device calculates the drug injection condition based on user input data received from a user terminal and sets an injection sequence corresponding to the drug injection condition,
the user input data includes a blood sugar level of a user and any one of activity information and meal information of the user, and
the drug injection condition includes any one of a drug injection amount, a drug injection period and a drug injection speed, and the drug injection amount and the drug injection period.

27. The drug injection method of the drug injection device of claim 16, wherein,
in the setting of the injection sequence, the drug injection device calculates the drug injection condition based on user biometric data received from an external measurement device and sets an injection sequence corresponding to the drug injection condition,
the user biometric data includes any one of user blood sugar level information and user activity information, and
the drug injection condition includes any one of a drug injection amount, a drug injection period and a drug injection speed, and the drug injection amount and the drug injection period.

28. The drug injection method of the drug injection device according to any one of claims 25 to 27, wherein,
in the setting of the injection sequence, the injection sequence corresponding to the drug injection condition is set by referring to a table in which multiple injection sequences are stored for each drug injection condition.

29. The drug injection method of the drug injection device of claim 16, wherein,
in the setting of the injection sequence, the drug injection device receives an injection sequence set by any one of a user terminal and a user input/output interface module, and sets a received injection sequence as the injection sequence.

30. A non-transitory computer-readable recording medium in which a computer program for performing the drug injection method according to claim 16 is stored.
